**Europäisches Patentamt**

**European Patent Office**

**Office européen des brevets**

(19)

(11) Veröffentlichungsnummer : **0 127 783 B2**

(12) # NEUE EUROPÄISCHE PATENTSCHRIFT

(45) Veröffentlichungstag der neuen Patentschrift :
28.08.91 Patentblatt 91/35

(51) Int. Cl.⁵ : **C07C 409/24, C07C 407/00**

(21) Anmeldenummer : **84105014.9**

(22) Anmeldetag : **04.05.84**

(54) Verfahren zum Phlegmatisieren von wasserunlöslichen Peroxycarbonsäuren.

(30) Priorität : 07.06.83 DE 3320496

(43) Veröffentlichungstag der Anmeldung :
12.12.84 Patentblatt 84/50

(45) Bekanntmachung des Hinweises auf die
Patenterteilung :
28.01.87 Patentblatt 87/05

(45) Bekanntmachung des Hinweises auf die
Entscheidung über den Einspruch :
28.08.91 Patentblatt 91/35

(84) Benannte Vertragsstaaten :
AT BE CH DE FR GB IT LI LU NL SE

(56) Entgegenhaltungen :
EP-B- 45 290
DE-A- 2 930 546
GB-A- 1 156 240

(56) Entgegenhaltungen :
GB-A- 2 032 421
US-A- 3 770 818
US-A- 4 233 235
Gmelins Handbuch der Anorganischen Chemie, 8 Aufl.Nr. 21 Ergänzungsband s.
1114(1966)

(73) Patentinhaber : Degussa Aktiengesellschaft
Weissfrauenstrasse 9
W-6000 Frankfurt am Main 1 (DE)
Patentinhaber : Henkel
Kommanditgesellschaft auf Aktien
Postfach 1100 Henkelstrasse 67
W-4000 Düsseldorf-Holthausen (DE)

(72) Erfinder : Dankowski, Manfred, Dr.
Stifterstrasse 14
W-8757 Karlstein (DE)
Erfinder : Hofen, Willi
Südring 54
W-6458 Rodenbach (DE)

EP 0 127 783 B2

EP 0 127 783 B2

## Beschreibung

Feste, wasserunlösliche Peroxycarbonsäuren sind in reinem oder hochkonzentriertem Zustand thermisch und mechanisch sensibel. Zur sicheren Handhabung dieser Verbindungen sind in der Literatur (D. Swern: Organic Peroxides Vol. I-III, 1970-72; R. Criegee in Houben-Weyl, Band 8) verschiedene Methoden beschrieben. Eine Phlegmatisierung kann unter Umständen auch durch den Zusatz von Alkali-, Erdalkali- und Ammoniumsalzen starker Mineralsäuren, wie z.B. schwefelsäure, geschehen.

Ein anderes Verfahren bildete das Phlegmatisierungsmittel in situ aus, im Anschluss an die Percarbonsäureherstellung aus wasserunlöslicher Carbonsäure und wässrigem Wasserstoffperoxid in Gegenwart von Schwefelsäure, und zwar durch Neutralisieren der nach der Reaktion im Gemisch vorhandenen Schwefelsäure mit Alkali- oder Erdalkalihydroxiden bzw. mit Alkalialuminaten, siehe US-PS 4 287135; DE-OS 2 930 546.

Es ist weiter ein kontinuierliches Verfahren zur Herstellung von Peroxycarbonsäuren, die nur wenig oder gar nicht wasserlöslich sind, bekannt, bei dem in Gegenwart eines sog. Lösungsmittel-Katalysators, vor allem konzentrierter Schwefelsäure, gearbeitet wird, der die wasserunlösliche Carbonsäure löst. Die gebildete, in Schwefelsäure unlösliche Percarbonsäure wird in einem organischen Lösungsmittel, das bei der Reaktion anwesend ist, aufgenommen. Die Neutralisation nimmt man im Anschluss an die Herstellung der Peroxycarbonsäure bei schwach erhöhter Temperatur mit Borax und Soda unter Bildung von Borsäure und Natriumsalzen vor, siehe EU-OS 0045290. Dieses Verfahren ist technisch aufwendig wegen der unabdinglichen Anwesenheit eines organischen Lösungsmittels, aus dem die darin gelöste Peroxycarbonsäure erst durch spezielle Massnahmen, wie Kristallisation bzw. Abdestillieren des Lösungsmittels, gewonnen werden kann.

Aufgabe der Erfindung ist daher ein Verfahren, das technisch einfach durchführbar ist und bei dem die Peroxycarbonsäuren in guten Ausbeuten und sehr gutem phlegmatisierten Zustand erhalten werden.

Es wurde nun gefunden, dass sich diese Aufgabe bei der Umsetzung wasserunlöslicher Carbonsäuren mit wässrigem Wasserstoffperoxid in Gegenwart von Schwefelsäure lösen lässt, wenn man diese Umsetzung bei Temperaturen ab dem Umwandlungspunkt des Natriumsulfatdekahydrates zu Thenardit bis 60°C durchführt, worauf man - gegebenenfalls nach Abkühlen des Reaktionsgemisches auf höchstens die genannte Umwandlungstemperatur von Natriumsulfatdekahydrat zu Thenardit - zunächst fertige wäßrige Natriumsulfatlösung der Reaktionsmischung zusetzt und dann die im Reaktionsgemisch vorhandene Schwefelsäure mit solchen Mengen an Natriumhydroxid in situ zu Natriumsulfat umsetzt, daß ein pH-Wert von 2 bis 6 resultiert, wonach man das erhaltene Reaktionsprodukt aus der Reaktionsmischung abtrennt und trocknet.

Der Umwandlungspunkt von Natriumsulfatdekahydrat zu Thenardit d.h. dem kristallwasserfreien Natriumsulfat, wird z.B. mit 32,4°C angegeben, siehe ULLMANN, Enzyklopädie der technischen Chemie, dritte Auflage, 1960, Bd. 12, S. 682. An anderer Stelle findet sich dieser Punkt bei 32,38°C, siehe D'Ans-Lax, 3. Aufl., 1-462.

Bevorzugte Umsetzungstemperaturen sind 45-60°C, wonach die Abkühlung bevorzugt auf Temperaturen von 40 auf 32,4°C, gegebenenfalls auf 32°C, erfolgt.

Bei der letzteren Temperatur wird das Natriumhydroxid zugesetzt. Das Natriumhydroxid kann in Lösung in beliebiger Konzentration zugefügt werden. In Frage kommen wässrige Lösungen von 20 bis 50 Gew.-%, bevorzugt 30 Gew.-%. Es wird, wie gesagt, so viel Natriumhydroxidlösung zugesetzt, dass die Reaktionsmischung einen pH-Wert von 2-6, bevorzugt 3-4, aufweist.

Als wasserunlösliche Carbonsäuren kommen aliphatische Carbonsäuren mit einer Gesamtkohlenstoffzahl von 6-16 C-Atomen oder aromatische Carbonsäuren mit einer Gesamtkohlenstoffzahl von 7-9 C-Atomen in Frage.

Aliphatische Carbonsäuren mit einer geringeren Kohlenstoffatomzahl als 6 sind schon recht wasserlöslich, aliphatische Carbonsäuren mit einer höheren Gesamtzahl an Kohlenstoffatomen als 16 sind auf Grund ihrer sehr stark hydrophoben Eigenschaften schlecht zu handhaben und häufig auch nicht handelsüblich.

Die Carbonsäuren können sowohl als Mono- wie Dicarbonsäuren eingesetzt werden. Besonders geeignet sind aliphatische Dicarbonsäuren mit einer Gesamtzahl an Kohlenstoffatomen von 9-13 C-Atomen, vor allem Azelainsäure, Dodcandisäure und Brassylsäure. Besonders bevorzugt ist Dodekandisäure.

Bei den aromatischen Carbonsäuren erwiesen sich Benzoesäure und die Phthalsäuren als besonders günstig. Wasserstoffperoxid wird in wässrigen Lösungen von 30 - 90 Gew.-%, bevorzugt 40 - 50 Gew.-%, verwendet. Schwefelsäure setzt man in Konzentrationen von 20 - 98 Gew.-%, bevorzugt 90 - 98 Gew.-%, ein, und zwar ebenfalls als wässrige Lösung.

Das erhaltene, phlegmatisierte Reaktionsprodukt lässt sich in üblicher Weise durch Filtrieren oder Zentrifugieren aus der Umsetzungsmischung abtrennen und wird in bekannter Weise getrocknet. Das Trocknen der Percarbonsäureprodukte wird durch das erfindungsgemässe Verfahren erleichtert.

Wird die Herstellung der Percarbonsäure nach dem erfindungsgemässen Verfahren vorgenommen und die in situ-Bildung von Natriumsulfat erfolgt bei Temperaturen ab mindestens dem Umwandlungspunkt von Natriumsulfatdekahydrat zu wasserfreiem Natriumsulfat (Thenardit), so bereitet die anschliessende Trocknung

2

des aus der Reaktionsmischung abgetrennten Produkts keine Schwierigkeiten mehr. Die Restfeuchte des abgetrennten, bevorzugt zentrifugierten Produktes ist hierdurch um etwa 50 % verringert.

Nach der Beendigung der Umsetzung zwischen Carbonsäure und Wasserstoffperoxid, aber noch vor der in situ-Bildung des Natriumsulfats, wird Natriumsulfat als wässrige Lösung dem Reaktionsgemisch zugesetzt. Anschliessend erfolgt dann die in situ-Bildung. Bevorzugt wird das direkt zugeführte Natriumsulfat bei der Temperatur zugesetzt, bei der auch die in situ-Bildung vorgenommen wird.

Auf diese Weise kann die für das Endprodukt gewünschte Menge an Natriumsulfat schon vor Abtrennung des Reaktionsproduktes vorhanden sein, so dass auch dieses Natriumsulfat in der Trockenphase in wasserfreier Form vorliegt. Die Trocknung wird bei 30 bis 60°C vorgenommen.

Der technische Fortschritt des erfindungsgemässen Verfahrens liegt darin, dass die Herstellung und Phlegmatisierung mit Natriumsulfat des Percarbonsäureproduktes einstufig und ohne Schwierigkeiten in der Trocknungsstufe erfolgt, und zwar in rein wässrigem Medium. Das Einsetzen von organischen Lösungsmitteln und deren aufwendige Wiedergewinnung fällt fort. Die Percarbonsäuregehalte in den erhaltenen Produkten sind hoch und entsprechen z.B. denen, die nach dem Verfahren der DE-OS 2 930 546 erhalten werden.

Die Erfindung wird anhand der nachfolgenden Beispiele erläutert.

Hierbei bedeuten:

DPDA = Diperoxydodecandisäure
AO    = Aktivsauerstoffgehalt
%     = Gewichtsprozent.

## Beispiel 1

In eine Oxydationsmischung, bestehend aus 170 g Wasserstoffperoxid (50 gew.-%ig) und 204 g Schwefelsäure (96 gew.-%ig) werden 115 g Dodecandisäure eindosiert und 8 h unter Rühren auf 50°C erhitzt. Nach dem Abkühlen auf 40°C wird der Ansatz bei dieser Temperatur mit 526 g Natriumsulfatlösung (30 gew.-%ig) versetzt und anschliessend mit 499 g Natriumhyxdroxidlösung (30 gew.-%ig) bis zum Erreichen des pH-Wertes von 3,5 neutralisiert. Anschliessend wird zentrifugiert und getrocknet. Trocknung bei 40°C.

Die Ausbeute an Persäure beträgt:              114,9 g ≙ 87,7% d.Th.
An Gesamt-AO-Gehalt wird gefunden:        9,0%
Die Dodecandisäurebilanz beträgt:         96,9%
Der Gehalt an DPDA beträgt:               72,7%.

## Beispiel 2

In eine Oxydationsmischung, bestehend aus 425 g Wasserstoffperoxid (50 gew.-%ig) und 510 g Schwefelsäure (96 gew.-%ig) werden 288 g Dodecandisäure eindosiert und 8 h unter Rühren auf 50°C erhitzt. Nach dem Abkühlen auf 40°C wird der Ansatz bei dieser Temperatur mit 1315 g Natriumsulfatlösung (30 gew.-%ig) versetzt und anschliessend mit 1288 g Natriumhydroxidlösung (30 gew.-%ig) bis zum Erreichen des pH-Wertes von 3,5 neutralisiert und mit 400 g Natriumsulfat konditioniert. Anschliessend wird zentrifugiert und getrocknet. Trocknung bei 40°C.

Die Ausbeute an Persäure beträgt:         255,7 g ≙ 78,0% d.Th.
An Gesamt-AO-Gehalt wird gefunden:        4,35%
Die Dodecandisäurebilanz beträgt:         96,8%
Der Gehalt an DPDA beträgt:               34,7%.

## Beispiel 3

In eine Oxydationsmischung, bestehend aus 102 g Wasserstoffperoxid (50 gew.-%ig), 204 g Schwefelsäure (96 gew.-%ig) und 25 g Natriumsulfatlösung (13 gew.-%ig), werden 115 g Dodecandisäure eindosiert und 8 h unter Rühren auf 60°C erhitzt. Nach dem Abkühlen auf 40°C wird der Ansatz bei dieser Temperatur mit 526 g Natriumsulfatlösung (30 gew.-%ig) versetzt und anschliessend mit 518 g Natriumhydroxidlösung (30 gew.-%ig) bis zum Erreichen des pH-Wertes von 3,5 neutralisiert und mit 160 g Natriumsulfat konditioniert. Anschliessend wird zentrifugiert und getrocknet. Trocknung bei 40°C.

EP 0 127 783 B2

Die Ausbeute an Persäure beträgt: 116,9 g ≙ 89,2% d.Th.
An Gesamt-AO-Gehalt wird gefunden: 4,36%
Der Gehält an DPDA beträgt: 35,1%.

*Beispiel 4*

In eine Oxidationsmischung, bestehend aus 102 g Wasserstoffperoxid (50 gew.-%ig), 204 g schwefelsäure (96 gew.-%ig) und 25 g Natriumsulfatlösung (13 gew.-%ig), werden 115 g Dodecandisäure eindosiert und 6 h unter Rühren auf 60°C erhitzt. Nach dem Abkühlen auf 40°C wird der Ansatz bei dieser Temperatur mit 526 g Natriumsulfatlösung (30 gew.-%ig ) versetzt und anschliessend mit 521 g Natriumhydroxidlösung (30 gew.-%ig) bis zum Erreichen des pH-Wertws von 3,5 neutralisiert und mit 160 g Natriumsulfat konditioniert. Anschliessend wird separiert und getrocknet. Trocknung bei 40°C.

Die Ausbeute an Persäure beträgt: 117,9 g ≙ 91,0% d.Th.
An Gesamt-AO-Gehalt wird gefunden: 4,29%
Die Dodecandisäurebilanz beträgt: 97,8%
Der Gehalt an DPDA beträgt: 53,1%.

*Beispiel 5*

In eine Oxidationsmischung, bestehend aus 102 g Wasserstoffperoxid (50 gew.-%ig), 204 g schwefelsäure (96 gew.-%ig) und 25 g Natriumsulfatlösung ( 13 gew.-%ig), werden 115 g Dodecandisäure eindosiert und 4 h unter Rühren auf 60°C erhitzt. Nach dem Abkühlen auf 40°C wird der Ansatz bei dieser Temperatur mit 526 g Natriumsulfatlösung (30 gew.-%ig) versetzt und anschliessend mit 521 g Natriumhydroxidlösung (30 gew.-%ig) bis zum Erreichen des pH-Wertes von 3,5 neutralisiert und mit 160 g Natriumsulfat konditioniert. Trocknung bei 40°C.

Die Ausbeute an Persäure beträgt: 117 g ≙ 98,3% d.Th.
An Gesamt-AO-Gehalt wird gefunden: 4,39%
Die Dodecandisäurebilanz beträgt: 97,2%
Der Gehalt an DPDA beträgt: 35,9%.

*Beispiel 6*

In eine Oxidationsmischung, bestehend aus 102 g Wasserstoffperoxid (50 gew.-%ig), 204 g schwefelsäure (96 gew.-%ig) und 25 g Natriumsulfatlösung (13 gew.-%ig), werden 115 g Dodecandisäure eindosiert und 2 h unter Rühren auf 60°C erhitzt. Nach dem Abkühlen auf 40°C wird der Ansatz bei dieser Temperatur mit 526 g Natriumsulfatlösung (30 gew.-%ig) versetzt und anschliessend mit 521 g Natriumhydroxid (30 gew.-%ig) bis zum Erreichen des pH-Wertes von 3,5 neutralisiert und mit 160 g Natriumsulfat konditioniert. Anschliessend wird separiert und getrocknet. Trocknung bei 40°C.

Die Ausbeute an Persäure beträgt: 113 g = 86,3% d.Th.
An Gesamt-AO-Gehalt wird gefunden: 4,28%
Die Dodecandisäurebilanz beträgt: 96,1%
Der gehalt an DPDA beträgt: 35,0%.

**Patentansprüche**

1. Verfahren zum Phlegmatisieren von wasserunlöslichen, aliphatischen oder aromatischen Peroxycarbonsäuren mit Hilfe von Natriumsulfat, dadurch gekennzeichnet, daß man die an sich bekannte Umsetzung

4

EP 0 127 783 B2

von wasserunlöslichen Carbonsäuren mit wäßrigem Wasserstoffperoxid in Gegenwart von Schwefelsäure zu den entsprechenden wasserunlöslichen Peroxycarbonsäuren bei Temperaturen ab dem Umwandlungspunkt des Natriumsulfatdekahydrats zu Thenardit bis 60 °C durchführt, worauf man - ggf. nach Abkühlen auf höchstens die genannte Umwandlungstemperatur von Natriumsulfatdekahydrat zu Thenardit - zunächst fertige wäßrige Natriumsulfatlösung der Reaktionsmischung zusetzt und dann die im Gemisch vorhandene Schwefelsäure mit solchen Mengen an Natriumhydroxid in situ zu Natriumsulfat umsetzt, daß ein pH-Wert von 2 bis 6 resultiert, wonach das erhaltene Reaktionsprodukt aus der Reaktionsmischung abgetrennt und getrocknet wird.

2. Verfahren nach Anspruch 1, dadurch gekennzeichnet, dass man die Umsetzung bei Temperaturen von 45 - 60°C durchführt und danach auf 40 - 32,4°C abkühlt und bei dieser Temperatur die in situ-Bildung von Natriumsulfat vornimmt.

3. Verfahren nach Anspruch 1 bis 3, dadurch gekennzeichnet, dass man im Anschluss an die in situ-Bildung von Natriumsulfat noch festes Natriumsulfat oder wässrige Natriumsulfatlösung in die Reaktionsmischung eingibt.

## Claims

1. A process for the desensitisation of water-insoluble, aliphatic or aromatic peroxy carboxylic acids by means of sodium sulphate, characterised in that the known reaction of water-insoluble carboxylic acids with aqueous hydrogen peroxide is carried out in the presence of sulphuric acid to form the corresponding water-insoluble peroxy carboxylic acids at temperatures from the conversion point of sodium sulphate decahydrate into thenardite to 60 °C, after which optionally after cooling to at most the abovementioned conversion temperature of sodium sulphate decahydrate into thenardite, finished aqueous sodium sulphate solution is firstly added to the reaction mixture and then the sulphuric acid present in the mixture is reacted in situ to form sodium sulphate using quantities of sodium hydroxide which are sufficient to produce a pH of from 2 to 6, whereupon the resultant reaction product is separated from the reaction mixture and dried.

2. A process according to Claim 1, characterized in that the reaction is carried out at temperatures of from 45 to 60 °C and is then cooled to 40 to 32,4 °C and the in situ formation of sodium sulphate is carried out at this temperature.

3. A process according to Claims 1 and 2, characterised in that still solid sodium sulphate or aqueous sodium sulphate solution is introduced into the reaction mixture after the in situ formation of sodium sulphate.

## Revendications

1. Procédé pour désensibiliser des acides percarboxyliques insolubles à l'eau, aliphatiques ou aromatiques, au moyen de sulfate de sodium, caractérisé en ce qu'il est effectué la réaction connue d'acides carboxyliques insolubles à l'eau avec de l'eau oxygénée, en présence d'acide sulfurique, formant les acides peroxycarboxyliques insolubles à l'eau correspondants à des températures partant de celle de la transformation du sulfate de sodium décahydraté en thénardite jusqu'à 60 °C, après quoi l'on ajoute d'abord, éventuellement après avoir refroidi à une température qui soit au maximum celle de la transformation mentionnée du sulfate de sodium décahydrate en thénardite, de la solution aqueuse de sulfate de sodium prepareé au mélange réactionnel après quoi l'on fait réagir in situ l'acide sulfurique présent dans le mélange en sulfate de sodium avec des quantités d'hydroxyde de sodium telles qu'on atteigne une valeur du pH de 2 à 6, après quoi le produit de la réaction du mélange réactionnel est séparé et séché.

2. Procédé suivant la revendication 1, caractérisé en ce quel'on effectue la réaction à des températures de 45 à 60 °C et ensuite refroidit à 40 à 32,4 °C, et assure, à cette température, la formation, in situ, de sulfate de sodium.

3. Procédé suivant les revendications 1 et 2, caractérisé en ce que, à la suite de la formation in situ, de sulfate de sodium, on introduit encore du sulfate de sodium solide, ou une solution aqueuse de sulfate de sodium dans le mélange réactionnel.